# EUROPEAN PATENT APPLICATION

(11) **EP 4 471 068 A1**
(43) Date of publication of application: **04.12.2024**
(21) Application number: 23746189.2
(22) Date of filing: 18.01.2023
(51) Int. Cl.: C07K 19/00, C12N 15/62, A61K 39/395, A61K 47/68, A61K 45/06, A61P 35/00, A61K 38/20

(54) **BIFUNCTIONAL MOLECULE FORMED BY FUSION OF PD1 ANTIBODY AND INTERLEUKIN 2**

(30) Priority: 30.01.2022 CN 202210114471
(71) Applicant: INSTITUTE OF BIOPHYSICS, CHINESE ACADEMY OF SCIENCES, Chaoyang District Beijing 100101 (CN)
(72) Inventor: PENG, Hua, Beijing 100101 (CN); CAO, Shuaishuai, Beijing 100101 (CN)
(74) Representative: Von Rohr Patentanwälte Partnerschaft mbB
(86) International application number: PCT/CN2023/072903
(87) International publication number: WO 2023/143308

(57) **Abstract**

The present invention relates to a bifunctional molecule formed by fusion of a PD1 antibody and interleukin 2, comprising: a heterodimer composed of a first monomer and a second monomer as follows: (1) a first monomer, formed by linking interleukin 2 (IL2) to an immunoglobulin Fc single chain; and (2) a second monomer, formed by linking an Fab/ScFv of an anti-T cell surface molecule antibody to an Fc single chain. The bifunctional molecule may also be a homodimer comprising a monomer comprising: (1) an interleukin 2 functional block; and (2) a monomer of an antibody or a monomer formed by linking an Fab/ScFv of an antibody to an Fc single chain, the antibody being an anti-T cell surface molecule antibody. The present invention further relates to use of the bifunctional molecule in the manufacture of anti-tumor medicament.

## Description

### FIELD OF THE INVENTION

The invention belongs to the field of biomedical technology, and specifically relates to a bifunctional molecule formed by fusion of PD1 antibody and interleukin 2.

### BACKGROUND OF THE INVENTION

IL-2 cytokine is a potent growth factor of T cells. It exerts its activity by binding with the IL-2 receptor (IL-2R) on the surface of T and NK cells, leading to its own phosphorylation through a JAK/STAT5-dependent pathway, and finally triggering the activation and proliferation of the corresponding cells (1). The exact mechanism by which IL-2 exerts its durable complete response (CR) remains controversial (2). The stimulating effect of IL-2 has been validated in multiple pathways required for the successful generation of adaptive and CTL-mediated anti-tumor responses (3). Following the recognition of antigens in MHC by T cell receptors (TCRs) and the binding of the co-stimulatory molecule CD28 to B7, IL-2 is considered as a third essential signal required for T cell clonal expansion and effector function (1, 3). Similarly, the function of CD8+ CTL is also heavily dependent on IL-2, as shown by their reduced effect or cytotoxic function in IL-2- or IL-2R-deficient mice (4-6). IL-2 increases the transport of CTL to extralymphatic sites of infection or tumors (1, 7). IL-2 is produced by Th1 cells in response to the activation of dendritic cells (DCs), which leads to the activation and proliferation of CD8 (3). A unique mechanism of IL-2 anti-tumor activity may be mediated by activating natural killer (NK) cells (8).

The efficacy of IL-2 in inducing durable complete response (CR) and partial response (PR) in patients with clear cell renal cell carcinoma (RCC) (9-11) was clinically validated in multiple phase II and phase III trials. In contrast, molecular targeted therapy failed to induce CR or cure cancer. Response rate (RR) (600,000-800,000 IU/kg q8h × 14 as tolerated) of IL-2 therapy reported in multiple phase III trials ranged from 20% to 23.2%, while CR ranged from 7% to 9% (9-11). Among patients who achieved CR, the efficacy was durable in most patients, with a median survival of more than 10 years (1, 15, 16). Alternative regimens and reduced doses of IL-2 were further tested, but did not show any improvement in efficacy (10-12).

Tumor microenvironment (TME) usually limits the efficacy of immunotherapy by increasing the production of regulatory T cells (Tregs) and/or decreasing T cell growth factors or their signaling. A major challenge is to provide sufficient cytokines to reactivate Cytotoxic T cells (CTLs) or inhibit Tregs. IL-2 is a "T cell growth factor", a pleiotropic cytokine that is produced upon antigen activation and plays a key role in immune response. IL-2 is a potent inducer of cytotoxic T cells and NK cells. Therefore, it is a sought-after treatment for various cancers. However, there are two main obstacles to the use of IL-2 in anti-cancer immunotherapy. Some T cells, such as Tregs, express heterotrimeric high-affinity receptors composed of CD25 (IL-2Rα), CD122 (IL-2Rβ) and CD132 (a common cytokine receptor γ chain) subunits. In contrast, immature CD8 T cells, CD4/CD8 memory T cells and NK cells express dimeric receptors (lacking the CD25 subunit) with low affinity (13, 14). When immature CD8 T cells are activated, they up-regulate the expression of CD25 (15). Therefore, Tregs can better compete with effector T cells for the use of IL-2. At present, IL-2 immunotherapy requires high-dose administration and multiple injections. In addition to the preferential expansion of Tregs, high dose of IL-2 may lead to vascular leakage syndrome, which may result in increased vascular permeability, hypotension, pulmonary edema, hepatocyte damage and renal failure (16-18).

Two fundamentally important strategies can be used to improve the use of IL-2 in immunotherapy: 1) how to remain active in tumor tissues while limiting systemic side effects; 2) how to preferentially activate effector T cells while limiting stimulation of Tregs. For the first issue, many research groups hope to reduce the toxicity of IL-2 by using antibody-based IL-2 delivery (19-24). For the second issue, some researchers have constructed IL-2 mutants which preferentially reduce their binding to CD25, resulting in better expansion of non-Treg population (25-27). Christopher Garcia's team constructed the IL-2 superkine (also called super-2) to eliminate the functional requirement of IL-2 for CD25 expression and increase the binding affinity for IL-2Rβ. Compared to IL-2, the IL-2 superkine induced super expansion of cytotoxic T cells, leading to improved antitumor responses *in vivo,* and elicited proportionally less expansion of T regulatory cells thereby reducing systemic side effects (26).

Whether a targeted IL-2 approach can improve the efficacy has been controversial. A recent study showed that F8-IL-2, an immune cytokine composed of F8 antibody fused to human IL-2, had a strong and improved inhibition of lymphoma progression compared to targeted IL-2 (21). Another recent study suggested that antigen specificity may not be important to the efficacy and biodistribution of immune cytokines (28, 29). They found that immunocytokine antigen specificity and Fcy receptor interactions did not seem necessary for therapeutic efficacy or biodistribution patterns because immunocytokines with irrelevant specificity and/or inactive mutant Fc domains behaved similarly to tumor-specific IL-2. They speculated that the biodistribution of IL-2 is mainly related to innate immune cells expressing IL-2R. We believe that the difference is related to the tumor model, the targeting ability of antibodies and the affinity between IL-2 and IL-2 receptors. Therefore, it is necessary to evaluate the targeting effect.

Here, we designed a bifunctional molecule (IL-2 linked to an antibody against the T cell surface antigen PD1) to target tumor infiltrating lymphocytes (TILs), because TILs express more T cell surface antigen PD1 than other cells. To reduce the binding of IL-2 to Tregs, we chose an IL-2 mutant (abII,2) with greatly reduced binding to IL-2α and IL-2β. We linked abIL2 to antibody against the T cell surface antigen PD1 (anti-PD1-abII,2) to increase its affinity for CD8+ T cells in tumors. Anti-PD1-abIL2 showed improved intratumoral T cell binding and potent anti-tumor effect. The PDL1 treatment resistance can also be overcome by using such bifunctional molecules.

Therefore, the anti-PD1-abIL2 bifunctional molecule is a novel and promising tumor treatment in clinic.

### SUMMARY OF THE INVENTION

The present invention firstly relates to a bifunctional molecule, which is a heterodimer, wherein
the heterodimer comprises:
   (1) a first monomer of the heterodimer, formed by linking interleukin 2 (IL2) to an immunoglobulin Fc single chain;
   (2) a second monomer of the heterodimer, formed by linking a Fab or ScFv of an anti-T cell surface molecule antibody to an immunoglobulin Fc single chain;
the first monomer and the second monomer are linked through dimerization of the Fc single chain to form the heterodimer;
the T cell surface molecule includes but is not limited to PD1, TIM-3, LAG-3, OX40, 4-1BB, ICOS and GITR;
the immunoglobulin Fc single chain is a natural immunoglobulin Fc single chain or an immunoglobulin Fc single chain in which ADCC effect is knocked out by gene mutation;
preferably, the immunoglobulin Fc single chain is a natural immunoglobulin Fc single chain or an immunoglobulin Fc single chain in which ADCC effect is knocked out by gene mutation; more preferably, the immunoglobulin Fc single chain is a human IgG Fc single chain.

The T cell surface molecule is PD1, and the anti-T cell surface molecule antibody is an anti-PD1 antibody (aPD1);
in the second monomer,
the Fab of the antibody is a Fab of a humanized antibody or a Fab of a fully human antibody;
the ScFv of the antibody is a ScFv of a humanized antibody or a ScFv of a fully human antibody;
more preferably, the second monomer is:
   a monomer of an anti-T cell surface molecule antibody, comprising a light chain and a heavy chain; preferably, the antibody is a humanized antibody or a fully human antibody.

More preferably, the heterodimer comprises:
(1) a first monomer, comprising sequentially from the N-terminus:
   1) a wild-type IL-2 protein having a sequence as shown in SEQ ID NO.1 or a mutant thereof comprising any one or any combination of mutations of R38L, F42A, D20K, R38A, F42K and K43E;
   2) an essential linker structure (G4S linker sequence), preferably, having a sequence as shown in SEQ ID NO.6;
   3) an IgG Fc single chain having a sequence as shown in SEQ ID NO.2, or a No-ADCC mutated IgG Fc having a sequence as shown in SEQ ID NO.3, or a knob mutant Fc having a sequence as shown in SEQ ID NO.4, or a hole mutant Fc having a sequence as shown in SEQ ID NO.5;
(2) a second monomer, comprising:
   1) an anti-PD1 antibody Fab region consisting of anti-PD1 antibody light chain VL-KCL having a sequence as shown in SEQ ID NO.7 and anti-PD1 antibody heavy chain VH&CH1 having a sequence as shown in SEQ ID NO.8; or
   2) an anti-PD1 single-chain antibody (ScFv) having a sequence as shown in SEQ ID NO.9; and
   3) an IgG Fc single chain having a sequence as shown in SEQ ID NO.2, or a No-ADCC mutated IgG Fc having a sequence as shown in SEQ ID NO.3, or a knob mutant Fc having a sequence as shown in SEQ ID NO.4, or a hole mutant Fc having a sequence as shown in SEQ ID NO.5;
   more preferably, the heterodimer comprises:
   a first monomer, which is a polypeptide having a sequence as shown in SEQ ID NO. 10 (abIL2-Fc) ;
   a second monomer, which is:
      (1) a second monomer consisting of an anti-PD1 antibody VH-CH1-Fc (knob) having a sequence as shown in SEQ ID NO. 11 and an anti-PD1 antibody light chain VL-KCL having a sequence as shown in SEQ ID NO.7; or
      (2) a polypeptide having a sequence as shown in SEQ ID NO. 12 (aPD1ScFv-Fc(knob)).

The present invention also relates to a bifunctional molecule, which is a homodimer, wherein,
a monomer of the homodimer is:
a monomer formed by linking a molecule of interleukin 2 (IL2) to a molecule of anti-PD1 antibody Fab by any means, or,
a monomer formed by linking a molecule of interleukin 2 (IL2) to a molecule of anti-PD1 single chain antibody (ScFv) by any means.

Preferably, the monomer of the homodimer comprises sequentially from the N-terminus:
(1) a wild-type IL-2 protein having a sequence as shown in SEQ ID NO.1 or a mutant thereof comprising any one or any combination of mutations of R38L, F42A, D20K, R38A, F42K and K43E;
(2) an essential linker structure (G4S linker sequence), preferably, having a sequence as shown in SEQ ID NO.6;
(3) a Fab or ScFv of an anti-PD1 antibody; the Fab is a Fab of a humanized antibody or a Fab of a fully human antibody, and the ScFv is a ScFv of a humanized antibody or a ScFv of a fully human antibody;
(4) an antibody Fc; the antibody Fc is a fully human wild-type Fc or a No-ADCC mutant Fc.

More preferably, the monomer of the homodimer has a sequence as shown in:
(1) SEQ ID NO.18 (aPD1-abIL2: VL-VH(ScFv)-Fc-abIL2);
(2) SEQ ID NO.19 (abIL2-aPD1: abIL2-VL-VH(ScFv)-Fc).

The present invention also relates to another bifunctional molecule, which is a bifunctional molecule comprising an anti-PD1 antibody (K) and abIL2, and the bifunctional molecule is a heterodimer;
the heterodimer comprises:
   (1) a first monomer, comprising sequentially from the N-terminus:
      1) a wild-type IL-2 protein having a sequence as shown in SEQ ID NO.1 or a mutant thereof comprising any one or any combination of mutations of R38L, F42A, D20K, R38A, F42K and K43E;
      2) an essential linker structure (G4S linker sequence), preferably, having a sequence as shown in SEQ ID NO.6;
      3) an IgG Fc single chain having a sequence as shown in SEQ ID NO.2, or a No-ADCC mutated IgG Fc having a sequence as shown in SEQ ID NO.3, or a knob mutant Fc having a sequence as shown in SEQ ID NO.4, or a hole mutant Fc having a sequence as shown in SEQ ID NO.5;
   (2) a second monomer, comprising:
      1) an antibody Fab region consisting of anti-PD1 antibody (K) light chain VL-KCL having a sequence as shown in SEQ ID NO. 13 and anti-PD1 antibody (K) heavy chain VH&CH1 having a sequence as shown in SEQ ID NO. 14; or
      2) an anti-PD1 single-chain antibody (K) (ScFv) having a sequence as shown in SEQ ID NO. 15; and
      3) an IgG Fc single chain having a sequence as shown in SEQ ID NO.2, or a No-ADCC mutated IgG Fc having a sequence as shown in SEQ ID NO.3, or a knob mutant Fc having a sequence as shown in SEQ ID NO.4, or a hole mutant Fc having a sequence as shown in SEQ ID NO.5;
more preferably, the heterodimer comprises:
   a first monomer, which is a polypeptide having a sequence as shown in SEQ ID NO. 10 (abIL2-Fc);
   a second monomer, which is:
      (1) a second monomer consisting of a polypeptide having a sequence as shown in SEQ ID NO. 16 (aPD1(K)VH-CH1-Fc (knob)) and an anti-PD1 antibody (K) light chain having a sequence as shown in SEQ ID NO. 13; or
      (2) a polypeptide having a sequence as shown in SEQ ID NO. 17 or SEQ ID NO.22 (aPD1(K) ScFv-Fc (knob)).

The present invention also relates to a bifunctional molecule, which is a homodimer, wherein,
a monomer of the homodimer is:
a monomer formed by linking a molecule of interleukin 2 (IL2) to a molecule of anti-PD1 antibody (K) Fab by any means, or,
a monomer formed by linking a molecule of interleukin 2 (IL2) to a molecule of anti-PD1 single-chain antibody (K) (ScFv) by any means.

Preferably, the monomer of the homodimer comprises sequentially from the N-terminus:
(1) a wild-type IL-2 protein having a sequence as shown in SEQ ID NO.1 or a mutant thereof comprising any one or any combination of mutations of R38L, F42A, D20K, R38A, F42K and K43E;
(2) an essential linker structure (G4S linker sequence), preferably, having a sequence as shown in SEQ ID NO.6;
(3) a Fab or ScFv of an anti-PD1 antibody (K); the Fab is a Fab of a humanized antibody or a Fab of a fully human antibody, and the ScFv is a ScFv of a humanized antibody or a ScFv of a fully human antibody;
(4) an antibody Fc; the antibody Fc is a fully human wild-type Fc or a No-ADCC mutant Fc.

More preferably, the monomer of the homodimer has a sequence as shown in:
(1) SEQ ID NO.20 (aPD1(K)-abIL2: VL-VH(ScFv)-Fc-abII,2),
(2) SEQ ID NO.21 (IL2-a PD1(K): II,2-VL-VH(ScFv)-Fc).

The present invention also relates to use of the bifunctional molecules:
(1) in the manufacture of an anti-tumor medicament;
(2) in the manufacture of an anti-tumor medicament used in combination with an immune checkpoint inhibitor;
(3) in the manufacture of an anti-tumor medicament that overcomes resistance to an immune checkpoint inhibitor;
(4) in the manufacture of an anti-tumor medicament used in combination with a T cell adoptive transfer;
(5) in the manufacture of an anti-tumor medicament that overcomes non-response to a T cell adoptive transfer.

Preferably, the immune checkpoint inhibitor is a PDL1 antibody;

Preferably, the T cell is an anti-tumor T cell; more preferably, the T cell is an anti-tumor CAR T cell or a structural analog thereof.

Beneficial effects of the invention:
(1) The present invention provides a corresponding solution to the existing bottleneck of clinical application of IL-2. Specifically, the abIL2/aPD1-hIgG1 antibody reduces the binding of IL2 to intratumoral Tregs while retaining the activation of IL2 on effector cells, thereby overcoming the adverse effect of Treg expansion caused by the use of IL2. These results provide a new idea for the clinical use of IL2;
(2) It is of great significance to overcome the resistance to immune checkpoint blockade therapy and the non-response to T cell adoptive transfer.

### BRIEF DESCRIPTION OF THE DRAWINGS

Fig. 1. Anti-PD1-abIL2 has lower *in vitro* activity.
Fig. 2. Anti-PD1-abIL2 can effectively control the growth of A20 tumor.
Fig. 3. Anti-PDL1-abII,2 cannot effectively control the growth of A20 tumor.
Fig. 4. The anti-tumor effect of anti-PD1-abIL2 is significantly better than that of anti-PDL1-abIL2.
Fig. 5. Anti-PD1-abIL2 can effectively control the growth of MC38 tumors.
Fig. 6. In A20 tumors, the anti-tumor effect of anti-PD1-abIL2 is significantly better than that of anti-PD1-II,2.
Fig. 7. In MC38 tumors, the anti-tumor effect of anti-PD1-abIL2 is significantly better than that of anti-PD1-II,2.
Fig. 8. The anti-tumor effect of anti-PD1-abIL2 does not depend on CD4 T cells.
Fig. 9. The anti-tumor effect of anti-PD1-abII,2 does not depend on NK cells.
Fig. 10. The anti-tumor effect of anti-PD1-abII,2 depends on CD8 T cells.
Fig. 11. Anti-hPD1-abIL2 can better control human tumors.
Fig. 12. Anti-PD1-abIL2 can act synergistically with T cell transplantation in tumor control.
Fig. 13. Anti-PD1-abIL2 can act synergistically with anti-PDL1 in tumor control.

### DETAILED DESCRIPTION OF THE EMBODIMENTS

### Experimental materials

### 1. Strains and plasmids

Strains: Top10 E.coli and DH5α E.coli competent cells (Beijing TransGen Biotech Co., Ltd.)

Plasmid:
pEE6.4-IgGκ-hIgG1, comprising the signal peptide of mouse IgGx and the Fc sequence of human IgG1, was used for the expression of antibodies.
pEE6.4-IgGκ-hlgG1-Fc-hole and pEE6.4-IgGx-hlgG1-Fc-knob were used for the expression of heterodimer proteins.
pEE6.4-PD1 VH-CH1-Fc-knob and pEE6.4-PD1 VL-CL were used for the expression of the antibody portion of heterodimer proteins; pEE6.4-abIL2-Fc-hole was used for the expression of the abIL2 portion of heterodimer proteins.

### 2. Laboratory animals

Wild-type C57BL/6, BALB/c and BALB/c-Rag mice were purchased from Beijing Vital River Laboratory Animal Center. Unless otherwise specified, female mice aged 8-10 weeks were used in all experiments. Mice were raised in a specific pathogen-free (SPF) barrier environment. Animal feeding and experimental operations complied with the relevant regulations of the Animal Management Committee of the Institute of Biophysics, Chinese Academy of Sciences.

### 3. Cell lines

MC38 cell line, a colorectal cancer cell line derived from a C57 mouse, was cultured in DMEM complete medium (containing 10% inactivated fetal bovine serum, 2 mmol/l L-glutamine, 0.1 mmol/l non-essential amino acids, 100U penicillin and 100 µg/ml streptomycin).

A20 cell line, a B cell lymphoma cell line derived from a BALB/c mouse, was cultured in RPMI1640 complete medium (containing 10% inactivated fetal bovine serum, 2 mmol/L L-glutamine, 0.1 mmol/L non-essential amino acid, 100U penicillin and 100 µg/ml streptomycin).

FreeStyleTM 293F cell line (Invitrogen), a suspension cell derived from HEK293 cell line, was cultured in SMM293-TII or CD OptiCHOTM medium and mainly used for transient transfection to express bifunctional molecules.

CTLL-2 cell lin, a murine T cell line, was used to detect the biological activity of IL2 and cultured in RPMI1640 complete medium (containing 10% inactivated fetal bovine serum, 2 mmol/L L-glutamine, 0.1 mmol/L non-essential amino acid, 100U penicillin, 100 µg/ml streptomycin, and 100 IU/ml recombinant IL2).

### Design and synthesis of genes and primers

The human wild-type IL2 gene sequences are shown in SEQ ID NO.1. The primers used in the experiment were designed by DNAMAN software and synthesized by Invitrogen.

**The proteins used in the following examples are all heterodimers, specifically:**
1. aPD1-abIL2 consists of the following first monomer and second monomer:
   (1) a polypeptide formed by fusing a Fc fragment to an IL2 containing mutations of R38L, F42A, D20K, R38A, F42K and K43E, having a sequence as shown in SEQ ID NO.10,
   (2) a second monomer of aPD1 having a sequence as shown in SEQ ID NO.22;
2. aPDL1-abIL2 consists of the following first monomer and second monomer:
   (1) a polypeptide formed by fusing a Fc fragment to an IL2 containing mutations of R38L, F42A, D20K, R38A, F42K and K43E, having a sequence as shown in SEQ ID NO.10,
   (2) a second monomer of aPDL1 having a sequence as shown in SEQ ID NO.23;
3. ahPD1-abIL2 consists of the following first monomer and second monomer:
   (1) a polypeptide formed by fusing a Fc fragment to an IL2 containing mutations of R38L, F42A, D20K, R38A, F42K and K43E, having a sequence as shown in SEQ ID NO. 10,
   (2) a second monomer of aPD1 having a sequence as shown in SEQ ID NO. 17;
4. aPDL1 antibody is commercialized atezolizumab consisting of:
   a first monomer : a polypeptide formed by fusing a Fc fragment to an IL2 containing mutations of R38L, F42A, D20K, R38A, F42K and K43E; a polypeptide having a sequence as shown in SEQ ID NO. 10 (abIL2-Fc) when involving a murine mode;
   a second monomer : an scFv-Fc of a PD1 antibody; when involving a murine mode, the polypeptide having a sequence as shown in SEQ ID NO. 10 (abIL2-Fc).

### Tumor inoculation and treatment in mice

### (1) Tumor inoculation and measurement:

### Tumor model establishment

5×10⁵-7.5×10⁵ MC38 and MC38-EGFR5 single cells were suspended in 100 µl PBS, and then inoculated subcutaneously on the back of C57BL/6 mice;
2×10⁶ A20 single cells were suspended in 100 µl PBS, and then inoculated subcutaneously on the back of BALB/c mice.

When a re-challenge experiment of the same tumor cells was performed on mice with tumor regression, the number of tumor cells inoculated was 5 times that of the initial tumor modeling, and the inoculation site was subcutaneous on the other side of the back of the mice. The tumor size was measured twice a week by using a vernier caliper to measure the long diameter (a), short diameter (b) and height (c) of the tumor. The tumor volume of mice = a×b×c/2.

### (2) Treatment:

Antibodies or bifunctional molecules were injected intraperitoneally. Intratumoral administration was also used in some experiments. The specific dosage will be described in specific experiments.

### In vivo cell deletion in mice

### (1) Depletion of CD4+T cells and CD8+T cells:

200 µg GK1.5 or TIB210 antibody were injected intraperitoneally to deplete CD4+ T cells and CD8+ T cells on the day before IL2 or IL2 bifunctional molecular therapy, then injected every 3 days, and the number of injections was adjusted according to the treatment cycle. Depletion efficiency was detected by flow cytometry.

### (2) Depletion of NK cells:

20 µl of NK cell-depleting antibody were injected intraperitoneally to deplete NK cells on the day before IL2 or IL2 bifunctional molecular therapy. Depletion efficiency was detected by flow cytometry.

### Example 1. aPD1-abIL2 had a lower ability to activate receptors, thereby avoiding peripheral side effects

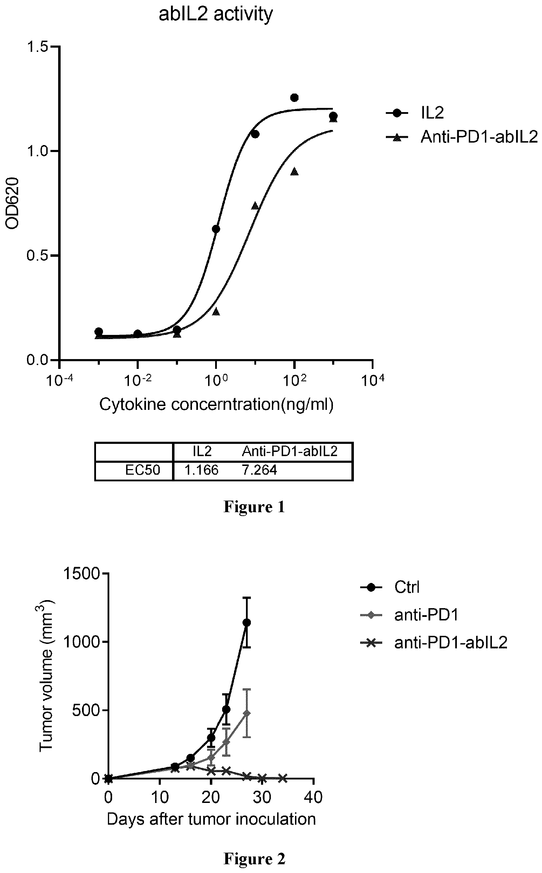

To reduce the toxicity of IL2, the binding ability of IL2 to IL2Rα and IL2Rβ was reduced. The design was then validated by *in vitro* CTLL2 proliferation experiments. CTLL2 cells were added with different concentrations of IL-2 or aPD1-abIL2, cultured for 72h and detected for the proliferation levels under different concentrations of IL2 or aPD1-abIL2 by CCK8 kit. The results showed that aPD1-abIL2 had a lower ability to expand CTLL2 cells (**Fig. 1**), indicating that the constructed antibody reduced IL2 activity.

### Example 2. aPD1-abIL2 bispecific antibody had a better anti-tumor activity

### 1. aPD1-abIL2 bispecific antibody had a significantly improved therapeutic effect compared with aPD1 antibody alone

BALB/c mice were subcutaneously inoculated with 2×10⁶ A20 tumor cells, and intraperitoneally injected with 10 µg aPD1 antibody or 20 µg aPD1-abIL2 antibody protein on Day 14 (D14) after tumor inoculation, respectively. Tumor size was measured twice a week. The results showed that aPD1-abIL2 bispecific antibody had a better therapeutic effect compared with aPD1 antibody alone (**Fig. 2**).

### 2. aPDL1-abIL2 bispecific antibody failed to significantly improve the therapeutic effect

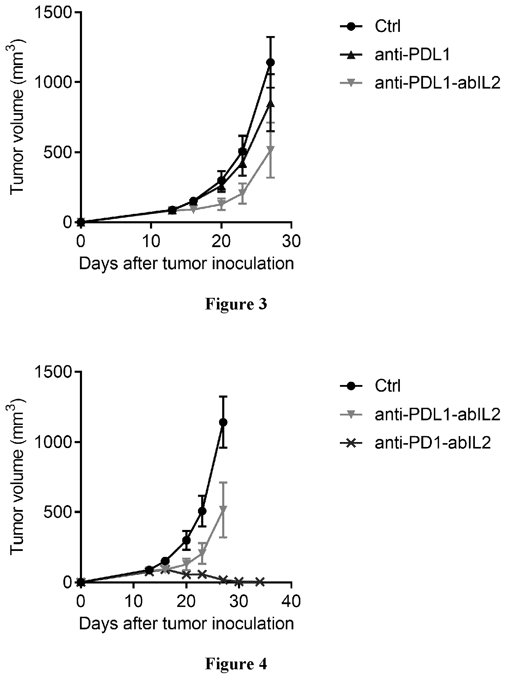

BALB/c mice were subcutaneously inoculated with 2×10⁶ A20 tumor cells, and intraperitoneally injected with 10 µg aPDL1 antibody or 20 µg aPDL1-abIL2 antibody protein on D14 after tumor inoculation, respectively. Tumor size was measured twice a week. The results showed that aPD1-abIL2 bispecific antibody did not have a better therapeutic effect compared with aPD1 antibody alone (**Fig. 3**).

### 3. aPD1-abIL2 bispecific antibody had a significantly improved therapeutic effect compared with aPDL1-abIL2 bispecific antibody

BALB/c mice were subcutaneously inoculated with 2×10⁶ A20 tumor cells, and intraperitoneally injected with 20 µg aPD1-abIL2 antibody or 20 µg aPDL1-abIL2 antibody protein on D14 after tumor inoculation, respectively. Tumor size was measured twice a week. The results showed that the aPD1-abIL2 bispecific antibody had a better therapeutic effect compared with aPDL1-abIL2 antibody alone (**Fig. 4**).

### 4. aPD1-abIL2 bispecific antibody had a significantly improved therapeutic effect compared with aPD1 antibody alone in MC38 colorectal cancer tumors

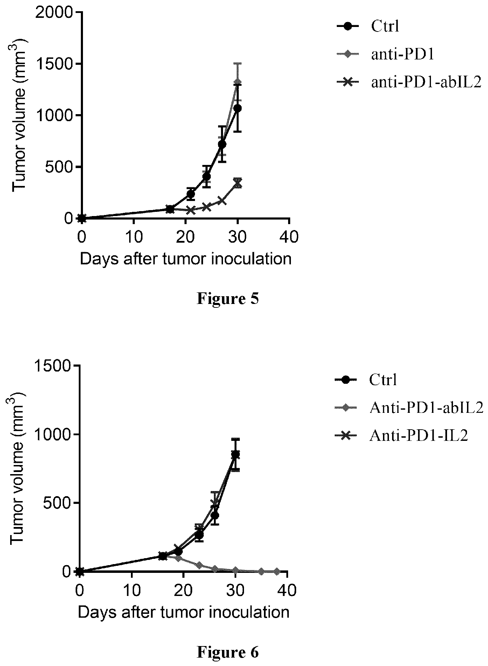

C57BL/6 mice were subcutaneously inoculated with 5×10⁵ MC38 tumor cells, and intraperitoneally injected with 10 µg aPD1 antibody or 20 µg aPD1-abIL2 antibody protein on Day 18 (D18) after tumor inoculation, respectively. Tumor size was measured twice a week. The results showed that aPD1-abIL2 bispecific antibody had a better therapeutic effect compared with aPD1 antibody alone (**Fig. 5**).

### 5. aPD1-abIL2 bispecific antibody had a significantly improved therapeutic effect compared with aPDL1-abIL2 bispecific antibody

BALB/c mice were subcutaneously inoculated with 2×10⁶ A20 tumor cells, and intraperitoneally injected with 20 µg aPD1-IL2 antibody or 20 µg aPDL1-abIL2 antibody protein on D14 after tumor inoculation, respectively. Tumor size was measured twice a week. The results showed that aPD1-abIL2 bispecific antibody had a better therapeutic effect compared with aPDL1-IL2 antibody alone (**Fig. 6**).

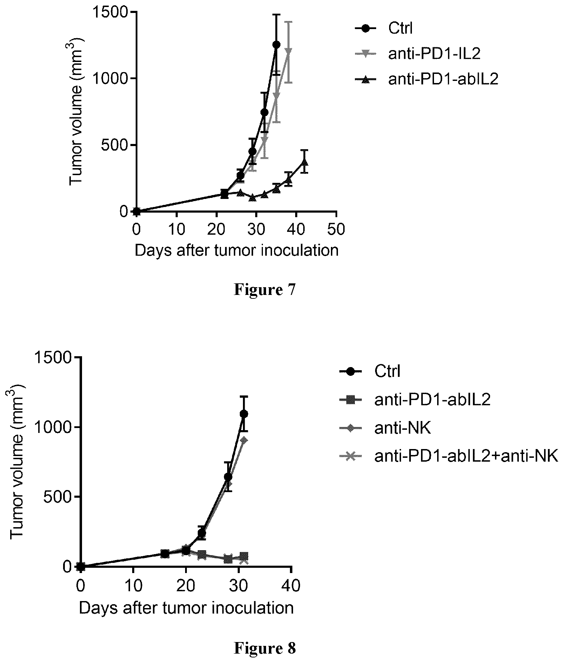

C57BL/6 mice were subcutaneously inoculated with 5×10⁵ MC38 tumor cells, and intraperitoneally injected with 20 µg aPD1-IL2 antibody or 20 µg aPD1-abIL2 antibody protein on D18 after tumor inoculation, respectively. Tumor size was measured twice a week. The results showed that aPD1-abIL2 bispecific antibody had a better therapeutic effect compared with aPD1-IL2 antibody alone (**Fig. 7**).

### Example 3. aPD1-abIL2 was capable of activating CD8 T cells

### 1. The therapeutic effect of aPD1-abIL2 bispecific antibody did not depend on NK cells

Since CD25 (IL2 receptor α) and PD1 were mainly expressed on activated effector T cells and NK cells, deletion experiments of different cell populations were performed separately to determine which population of immune cells the treatment of aPD1-abIL2 antibody mainly depended on.

BALB/c mice were subcutaneously inoculated with 2×10⁶ A20 tumor cells, intraperitoneally injected with 20 µg aPD1-abII,2 bifunctional molecule on day 17, and intraperitoneally injected with 20 µl of NK cell-depleting antibody one day before the treatment every 4 days for a total of 3 injections.

In the experiment, the aPD1-abIL2 antibody still had a therapeutic effect after the deletion of NK cells, indicating that NK cells were not the main effector cells for the therapeutic effect of the antibody (**Fig. 8**).

### 2. The therapeutic effect of aPD1-abIL2 antibody depended on CD8 T cells

The role of T cells in antibody therapy was further verified.

BALB/c mice were subcutaneously inoculated with 2×10⁶ A20 tumor cells and intraperitoneally injected with 20 µg aPD1-abIL2 protein on day 17 after tumor inoculation, along with the intraperitoneal injection of 200 µg of CD4 T cell depleting antibody (clone number: GK1.5, prepared in our laboratory), 200 µg of CD8 T cell depleting antibody (clone number: TIB210, prepared in our laboratory) or both for a total of 3 injections.

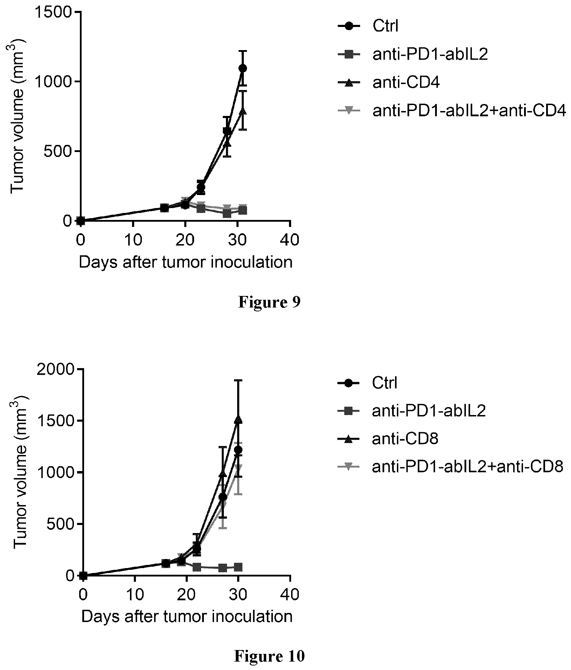

Depletion experiments of CD4 T and CD8 T cells showed that the therapeutic effect of antibody therapy was not significantly reduced after depleting CD4 T cells, but significantly reduced after depleting CD8 T cells; and completely disappeared after depleting both CD4 T and CD8 T cells. It indicated that the therapeutic effect of aPD1-abIL2 antibody depended on T cells, and mainly CD8 T cells (**Figs. 9 and Figs. 10**).

### Example 4. aPD1-abIL2 bispecific antibody had a better anti-tumor activity in humanized mice

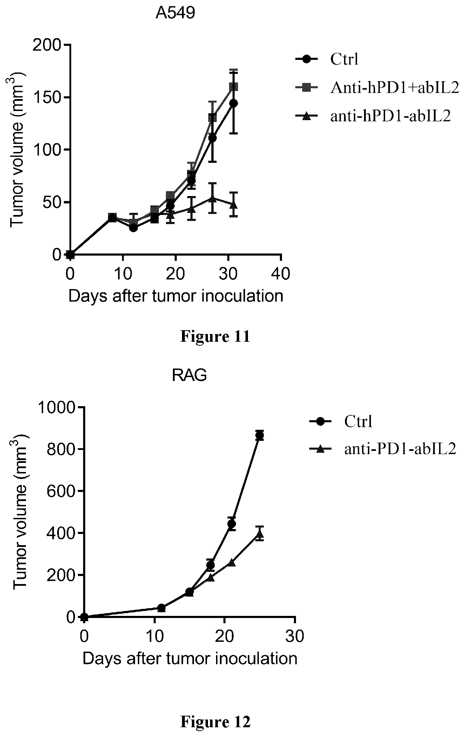

Humanized mice were subcutaneously inoculated with 2×10⁶ A549 tumor cells, and intraperitoneally injected with 10 µg ahPD1 antibody plus 10 µg abIL2, or 20 µg ahPD1-abII,2 antibody protein on D9 after tumor inoculation, respectively. The tumor size was measured twice a week. The results showed that ahPD1-abIL2 bispecific antibody had a better therapeutic effect compared with the combined antibody (**Fig. 11**).

### Example 5. aPD1-abIL2 was capable of overcoming non-response to T cell adoptive transfer therapy

Rag knockout mice were subcutaneously inoculated with 2×10⁶ A20-HA tumor cells, adoptively transferred with 2×10⁶ OTI cells 12 days later, and intraperitoneally injected with 20 µg aPD1-abIL2. The tumor size was measured twice a week. The results showed that aPD1-abIL2 bispecific antibody had a better therapeutic effect compared with the T cell adoptive transfer therapy alone (Fig. 12).

### Example 6. aPD1-abIL2 was capable of overcoming non-response to PDL1 antibody therapy

When the tumor of A20 tumor-bearing mice was below 150 mm³, aPD1-abIL2 antibody therapy alone had a good elimination effect. However, when the tumor was larger, antibody therapy alone could only control tumor growth but not completely eliminate it. To improve the therapeutic effect, bispecific antibodies and immune checkpoint inhibitory antibodies were combined to see whether the therapeutic effect can be improved.

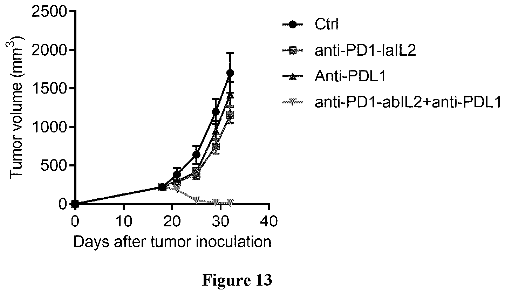

Specific protocol: BALB/c mice were subcutaneously inoculated with 2×10⁶ A20 tumor cells, and intraperitoneally injected with 50 µg aPDL1 antibody or/and 20 µg aPDL1-abIL2 antibody protein on D20 after tumor inoculation, respectively. Tumor size was measured twice a week. The results showed that the combination therapy had a better therapeutic effect compared with aPDL1 antibody or aPD1-abIL2 bispecific antibody alone (**Fig. 13**).

Finally, it should be noted that the above examples are only used to help those skilled in the art to understand the essence of the present invention, and are not intended to limit the protection scope of the present invention.

[References]

1.	Malek TR, and Bayer AL. Tolerance, not immunity, crucially depends on IL-2. Nature reviews
	Immunology. 2004;4(9):665-74.
2.	Rosenberg SA, Mule JJ, Spiess PJ, Reichert CM, and Schwarz SL. Regression of established
	pulmonary metastases and subcutaneous tumor mediated by the systemic administration of high-dose
	recombinant interleukin 2. The Journal of experimental medicine. 1985;161(5):1169-88.
3.	Murphy K, Travers P, and Walport M. In: Murphy K, Travers P, and Walport M eds. Janeway's
	Immunobiology. New York: Garland Science; 2008:323-79.
4.	Cousens LP, Orange JS, and Biron CA. Endogenous IL-2 contributes to T cell expansion and IFN-
	gamma production during lymphocytic choriomeningitis virus infection. J Immunol.
	1995;155(12):5690-9.
5.	Su HC, Cousens LP, Fast LD, Slifka MK, Bungiro RD, Ahmed R, et al. CD4+ and CD8+ T cell
	interactions in IFN-gamma and IL-4 responses to viral infections: requirements for IL-2. J Immunol.
	1998;160(10):5007-17.
6.	Blattman JN, Grayson JM, Wherry EJ, Kaech SM, Smith KA, and Ahmed R. Therapeutic use of IL-
	2 to enhance antiviral T-cell responses in vivo. Nature medicine. 2003;9(5):540-7.
7.	D'Souza WN, Schluns KS, Masopust D, and Lefrancois L. Essential role for IL-2 in the regulation of
	antiviral extralymphoid CD8 T cell responses. J Immunol. 2002;168(11):5566-72.

8.	Suzuki H, Duncan GS, Takimoto H, and Mak TW. Abnormal development of intestinal
	intraepithelial lymphocytes and peripheral natural killer cells in mice lacking the IL-2 receptor beta
	chain. The Journal of experimental medicine. 1997;185(3):499-505.
9.	Klapper JA, Downey SG, Smith FO, Yang JC, Hughes MS, Kammula US, et al. High-dose
	interleukin-2 for the treatment of metastatic renal cell carcinoma : a retrospective analysis of
	response and survival in patients treated in the surgery branch at the National Cancer Institute
	between 1986 and 2006. Cancer. 2008;113(2):293-301.
10.	McDermott DF, Regan MM, Clark JI, Flaherty LE, Weiss GR, Logan TF, et al. Randomized phase
	III trial of high-dose interleukin-2 versus subcutaneous interleukin-2 and interferon in patients with
	metastatic renal cell carcinoma. J Clin Oncol. 2005;23(1):133-41.
11.	Yang JC, Sherry RM, Steinberg SM, Topalian SL, Schwartzentruber DJ, Hwu P, et al. Randomized
	study of high-dose and low-dose interleukin-2 in patients with metastatic renal cancer. J Clin Oncol.
	2003;21(16):3127-32.
12.	Negrier S, Escudier B, Lasset C, Douillard JY, Savary J, Chevreau C, et al. Recombinant human
	interleukin-2, recombinant human interferon alfa-2a, or both in metastatic renal-cell carcinoma.
	Groupe Francais d'Immunotherapie. N Engl J Med. 1998;338(18):1272-8.
13.	Boyman O, and Sprent J. The role of interleukin-2 during homeostasis and activation of the immune
	system. Nature reviews Immunology. 2012;12(3):180-90.
14.	Liao W, Lin JX, and Leonard WJ. Interleukin-2 at the crossroads of effector responses, tolerance,
	and immunotherapy. Immunity. 2013;38(1):13-25.
15.	Moriggl R, Marine JC, Topham DJ, Teglund S, Sexl V, McKay C, et al. Differential roles of
	cytokine signaling during T-cell development. Cold Spring Harb Symp Quant Biol. 1999;64:389-95.
16.	Skrombolas D, and Frelinger JG. Challenges and developing solutions for increasing the benefits of
	IL-2 treatment in tumor therapy. Expert Rev Clin Immunol. 2014;10(2):207-17.
17.	Panelli MC, White R, Foster M, Martin B, Wang E, Smith K, et al. Forecasting the cytokine storm
	following systemic interleukin (IL)-2 administration. J Transl Med. 2004;2(1):17.
18.	Chavez AR, Buchser W, Basse PH, Liang X, Appleman LJ, Maranchie JK, et al. Pharmacologic
	administration of interleukin-2. Ann N Y Acad Sci. 2009;1182:14-27.
19.	Becker JC, Varki N, Gillies SD, Furukawa K, and Reisfeld RA. An antibody-interleukin 2 fusion
	protein overcomes tumor heterogeneity by induction of a cellular immune response. Proceedings of
	the National Academy of Sciences of the United States of America. 1996;93(15):7826-31.
20.	Gutbrodt KL, Casi G, and Neri D. Antibody-based delivery of IL2 and cytotoxics eradicates tumors
	in immunocompetent mice. Molecular cancer therapeutics. 2014;13(7):1772-6.
21.	Gutbrodt KL, Schliemann C, Giovannoni L, Frey K, Pabst T, Klapper W, et al. Antibody-based
	delivery of interleukin-2 to neovasculature has potent activity against acute myeloid leukemia.
	Science translational medicine. 2013;5(201):201ra118.
22.	Du YJ, Lin ZM, Zhao YH, Feng XP, Wang CQ, Wang G, et al. Stability of the recombinant
	antierbB2 scFvFcinterleukin2 fusion protein and its inhibition of HER2overexpressing tumor cells.
	International journal of oncology. 2013;42(2):507-16.
23.	Gillies SD, Lan Y, Hettmann T, Brunkhorst B, Sun Y, Mueller SO, et al. A low-toxicity IL-2-based
	immunocytokine retains antitumor activity despite its high degree of IL-2 receptor selectivity. Clin
	Cancer Res. 2011;17(11):3673-85.
24.	Yang RK, Kalogriopoulos NA, Rakhmilevich AL, Ranheim EA, Seo S, Kim K, et al. Intratumoral
	treatment of smaller mouse neuroblastoma tumors with a recombinant protein consisting of IL-2
	linked to the hu14.18 antibody increases intratumoral CD8+ T and NK cells and improves survival.
	Cancer immunology, immunotherapy : CII. 2013;62(8):1303-13.
25.	Carmenate T, Pacios A, Enamorado M, Moreno E, Garcia-Martinez K, Fuente D, et al. Human IL-2
	mutein with higher antitumor efficacy than wild type IL-2. J Immunol. 2013;190(12):6230-8.
26.	Levin AM, Bates DL, Ring AM, Krieg C, Lin JT, Su L, et al. Exploiting a natural conformational
	switch to engineer an interleukin-2 'superkine'. Nature. 2012;484(7395):529-33.
27.	Rosalia RA, Arenas-Ramirez N, Bouchaud G, Raeber ME, and Boyman O. Use of enhanced
	interleukin-2 formulations for improved immunotherapy against cancer. Current opinion in chemical
	biology. 2014;23:39-46.
28.	Tzeng A, Kwan BH, Opel CF, Navaratna T, and Wittrup KD. Antigen specificity can be irrelevant to
	immunocytokine efficacy and biodistribution. Proceedings of the National Academy of Sciences of
	the United States of America. 2015;112(11):3320-5.

29.	Zhu EF, Gai SA, Opel CF, Kwan BH, Surana R, Mihm MC, et al. Synergistic innate and adaptive
immune response to combination immunotherapy with anti-tumor antigen antibodies and extended
serum half-life IL-2. Cancer cell. 2015;27(4):489-501.

## Claims

1. A bifunctional molecule, which is a heterodimer, wherein,
the heterodimer comprises:
(1) a first monomer, formed by linking interleukin 2 (IL2) to an immunoglobulin Fc single chain;
(2) a second monomer, formed by linking a Fab or ScFv of an anti-T cell surface molecule antibody to an immunoglobulin Fc single chain;
the first monomer and the second monomer are linked through dimerization of the Fc single chain to form the heterodimer;
the T cell surface molecule includes but is not limited to PD1, TIM-3, LAG-3, OX40, 4-1BB, ICOS and GITR.

2. The bifunctional molecule according to claim 1, wherein the immunoglobulin Fc single chain is a natural immunoglobulin Fc single chain or an immunoglobulin Fc single chain in which ADCC effect is knocked out by gene mutation; preferably, the immunoglobulin Fc single chain is a human IgG Fc single chain.

3. The bifunctional molecule according to any one of claim 1, wherein,
in the second monomer,
the Fab of the antibody is a Fab of a humanized antibody or a Fab of a fully human antibody;
the ScFv of the antibody is a ScFv of a humanized antibody or a ScFv of a fully human antibody;
preferably, the second monomer is:
a monomer of an anti-T cell surface molecule antibody, comprising a light chain and a heavy chain;
more preferably, the antibody is a monomer of a humanized anti-T cell surface molecule antibody or a fully human anti-T cell surface molecule antibody, comprising a light chain and a heavy chain.

4. The bifunctional molecule according to any one of claims 1-3, wherein,
the T cell surface molecule is PD1,
the anti-T cell surface molecule antibody is an anti-PD1 antibody (aPD1).

5. The bifunctional molecule according to any one of claims 1-4, wherein,
the heterodimer comprises:
(1) a first monomer, comprising sequentially from the N-terminus:
1) a wild-type IL-2 protein having a sequence as shown in SEQ ID NO.1 or a mutant thereof comprising any one or any combination of mutations of R38L, F42A, D20K, R38A, F42K and K43E;
2) an essential linker structure (G4S linker sequence), preferably, having a sequence as shown in SEQ ID NO.6;
3) an IgG Fc single chain having a sequence as shown in SEQ ID NO.2, or an ADCC-knockout mutated IgG Fc having a sequence as shown in SEQ ID NO.3, or a knob mutant Fc having a sequence as shown in SEQ ID NO.4, or a hole mutant Fc having a sequence as shown in SEQ ID NO.5;
(2) a second monomer, comprising:
1) an anti-PD1 antibody Fab region consisting of anti-PD1 antibody light chain VL-KCL having a sequence as shown in SEQ ID NO.7 and anti-PD1 antibody heavy chain VH&CH1 having a sequence as shown in SEQ ID NO.8; or
an anti-PD1 single-chain antibody (ScFv) having a sequence as shown in SEQ ID NO.9; and,
2) an IgG Fc single chain having a sequence as shown in SEQ ID NO.2, or an ADCC-knockout mutated IgG Fc having a sequence as shown in SEQ ID NO.3, or a knob mutant Fc having a sequence as shown in SEQ ID NO.4, or a hole mutant Fc having a sequence as shown in SEQ ID NO.5;

6. The bifunctional molecule according to any one of claims 1-5, wherein,
the heterodimer comprises:
a first monomer, which is a polypeptide having a sequence as shown in SEQ ID NO.10 (abIL2-Fc);
a second monomer, which is:
(1) a Fab construct of an anti-PD1 antibody consisting of an anti-PD1 antibody light chain VL-KCL having a sequence as shown in SEQ ID NO.7 and an anti-PD1 antibody VH-CH1-Fc (knob) having a sequence as shown in SEQ ID NO. 11; or
(2) a polypeptide having a sequence as shown in SEQ ID NO.12 (aPD1ScFv-Fc(knob)).

7. The bifunctional molecule according to claim 5, wherein,
the second monomer comprises:
1) an antibody Fab region consisting of anti-PD1 antibody (K) light chain having a sequence as shown in SEQ ID NO. 13 and anti-PD1 antibody (K) heavy chain VH&CH1 having a sequence as shown in SEQ ID NO. 14; or
2) an anti-PD1 single-chain antibody (K) (ScFv) having a sequence as shown in SEQ ID NO. 15; and
3) an IgG Fc single chain having a sequence as shown in SEQ ID NO.2, or a No-ADCC mutated IgG Fc having a sequence as shown in SEQ ID NO.3, or a knob mutant Fc having a sequence as shown in SEQ ID NO.4, or a hole mutant Fc having a sequence as shown in SEQ ID NO.5.

8. The bifunctional molecule according to claim 6, wherein,
the second monomer is:
(1) a Fab construct of an anti-PD1 antibody consisting of a polypeptide having a sequence as shown in SEQ ID NO. 16 (aPD1(K)VH-CH1-Fc (knob)) and a variable region of an anti-PD1 antibody light chain having a sequence as shown in SEQ ID NO. 13; or
(2) a polypeptide having a sequence as shown in SEQ ID NO. 17 or SEQ ID NO.22 (aPD1(K) ScFv-Fc (knob)).

9. A bifunctional molecule, which is a homodimer, wherein,
the monomer of the homodimer is:
a monomer formed by linking a molecule of interleukin 2 (IL2) to a molecule of anti-PD1 antibody Fab by any means, or,
a monomer formed by linking a molecule of interleukin 2 (IL2) to a molecule of anti-PD1 single chain antibody (ScFv) by any means.

10. The bifunctional molecule according to claim 9, wherein,
the monomer of the homodimer comprises sequentially from the N-terminus:
(1) a wild-type IL-2 having a sequence as shown in SEQ ID NO.1 or a mutant thereof comprising any one or any combination of mutations of R38L, F42A, D20K, R38A, F42K and K43E;
(2) an essential linker structure (G4S linker sequence), preferably, having a sequence as shown in SEQ ID NO.6;
(3) a Fab/ScFv of an anti-PD1 antibody (aPD1); the Fab is a Fab of a humanized antibody or a Fab of a fully human antibody, and the ScFv is a ScFv of a humanized antibody or a ScFv of a fully human antibody;
(4) an antibody Fc; the antibody Fc is a fully human wild-type Fc or a No-ADCC mutant Fc.

11. The bifunctional molecule according to claim 9, wherein,
the monomer of the homodimer is:
(1) a polypeptide having a sequence as shown in SEQ ID NO.18 (aPD1-abIL2: aPD1(ScFv)-Fc-abIL2), or
(2) a polypeptide having a sequence as shown in SEQ ID NO.19 (abIL2-aPD1: abIL2-aPD1(ScFv)-Fc), or
(3) a polypeptide having a sequence as shown in SEQ ID NO.20 (aPD1(K)-abIL2: aPD1(K)(ScFv)-Fc-abIL2), or
(4) a polypeptide having a sequence as shown in SEQ ID NO.21 (IL2-aPD1(K):IL2-aPD1(K)(ScFv)-Fc).

12. Use of the bifunctional molecule according to any one of claims 1-11
(1) in the manufacture of an anti-tumor medicament;
(2) in the manufacture of anti-tumor medicament used in combination with an immune checkpoint inhibitors;
(3) in the manufacture of an anti-tumor medicament that overcomes resistance to an immune checkpoint inhibitors;
(4) in the manufacture of an anti-tumor medicament used in combination with a T cell adoptive transfer;
(5) in the manufacture of an anti-tumor medicament that overcomes non-response to a T cell adoptive transfer.

13. The use according to claim 12, wherein, the immune checkpoint inhibitor is an anti-PDL1 antagonist; preferably, the anti-PDL1 antagonist is an anti-PDL1 antibody.

14. The use according to claim 12, wherein, the T cell is an anti-tumor T cell; more preferably, the T cell is an anti-tumor CAR T cell or a structural analog thereof.

15. A medicament or a pharmaceutical composition comprising the bifunctional molecule of any one of claims 1-12.
